# EUROPÄISCHE PATENTSCHRIFT

(11) **EP 2 467 274 B1**
(45) Veröffentlichungstag und Bekanntmachung des Hinweises auf die Patenterteilung: **29.07.2015**
(21) Anmeldenummer: 10742476.4
(22) Anmeldetag: 06.08.2010
(51) Int. Cl.: B60H 1/00, B60H 3/00

(54) **LUFTFÜHRUNGSKANAL FÜR IONISIERUNGSVORRICHTUNG**
AIR DUCT FOR IONISATION DEVICE
CONDUIT D'AIR POUR APPAREIL D'IONISATION

(30) Priorität: 21.08.2009 DE 102009038298
(43) Veröffentlichungstag der Anmeldung: 27.06.2012
(73) Patentinhaber: MAHLE Behr GmbH & Co. KG, 70469 Stuttgart (DE)
(72) Erfinder: RAIS, Thomas, 71672 Marbach/Neckar (DE); PITZ, Eric, 70199 Stuttgart (DE)
(74) Vertreter: Grauel, Andreas
(86) Internationale Anmeldenummer: PCT/EP2010/061500
(87) Internationale Veröffentlichungsnummer: WO 2011/020717

(56) Entgegenhaltungen:
- EP-A1- 1 323 589
- WO-A1-2006/134681
- DE-A1-102005 054 961
- JP-A- 2002 277 010
- JP-A- 2004 079 471
- JP-A- 2004 182 123

## Beschreibung

Die Erfindung betrifft eine Luftfühtungsvorrichtung, insbesondere einen Luftführungskanal und/oder eine Luftaustassdüse, bevorzugt eine Luftführungsvorrichtung für eine Ionisierungsvorrichtung. Die Erfindung betrifft weiterhin eine Ionteierungsvorrichtung sowie eine Klimaanlage, insbesondere eine Kraftfahrzeugkilmaanlage.

Um die im Zusammenhang mit der Belüftung von Kraftfahrzeugen über die Jahre hinweg gestiegenen Komfortanforderungen der Fahrzeugkunden zu befriedigen, hat die Funktionsvielfalt und die Komplexität von Kraftfahrzeugklimaanlagen entsprechend zugenommen.

So haben sich Klimaanlagen zwischenzeitlich in sämtlichen Fahrzeugklassen durchgesetzt. Mit derartigen Fahrzeugkilmaanlagen kann die dem Fahrzeuginnenraum zuzuführende Frischluft erwärmt werden. Heutzutage ist jedoch meist auch eine Kühlfunkflon vorgesehen, mit der die dem Fahrzeuginnenraum zuzuführende Frischluft auch abgekühlt werden kann.

Weitere Funktionen, die zwischenzeitlich in zunehmendem Umfang bei der Belüftung von Kraftfahrzeugen realisiert werden, sind beispielsweise Filter, die die dem Fahrzeuginnenraum zuzuführende Frischluft reinigen und/oder zur Entfernung von unerwünschten Gerüchen (insbesondere Außenluftgerüchen) eingesetzt werden.

Seit einiger Zeit werden in Kraftfahrzeugen auch im Rahmen der Eratausrüstung Ionisierungsgeräte verbaut. Die Ionisierungsgeräte sollen einerseits für eine Luftauffrischung sorgen, also für ein Empfinden, dass eine "frische Luft" vorliegt. Andererseits werden Ionisierungsgeräte dazu verwendet, um Krankheitserreger (insbesondere Bakterien und/oder Viren), welche sich gegebennenfalls in der dem Fahrzeuginnenraum zuzuführenden Frischluft und/oder Umluft befinden, abzutöten. Um Krankheitserreger abzutöten wird meist eine Kombination aus einer positiv geladenen Elektrode sowie einer negativ geladenen Elektrode verwendet. Als wirksames Ion dient dabei häufig HO₂⁻. Um HO₂⁻ zu erzeugen wird aus Wasser (H₂O), welches In Form von Luftfeuchtigkeit vorliegt mit Hilfe einer positiv geladenen Elektrode H⁺ erzeugt, weiches anschließend an der negativ geladenen Elektrode zu H reduziert wird. H kombiniert anschließend mit O₂⁻ zum wirksamen HO₂⁻.

Zur Lufterfrischurg werden dagegen meist negativ geladenen (einfache) Hochspannungselektroden verwendet, welche zum Beispiel in der zuzuführenden Luft enthaltene Sauerstoffmoleküle (O₂) zu negativ geladenen Sauerstoffionen reduzieren (O₂⁻).

Damit die Ionisierungsgeräte ihre Wirksamkeit entfalten können, ist es nicht nur erforderlich, dass diese Ionen erzeugen, sondern dass die von den Ionisierungsgeräten erzeugten Ionen auch über eine längere Zeitdauer hinweg vorhanden bleiben und in aller Regel auch in den zu belüftenden Innenraum freigesetzt werden. Dies ist insbesondere im Kraftfahrzeugbereich ein Problem, wo die aufbereitete Luft über eine größere Anzahl von Luftleitelementen, Luftleitklappen und Auslaßdüsen gelenkt wird, bevor die aufbereitete Luft in den Kraftfahrzeuginnenraum abgegeben wird. Versuche haben ergeben, dass sich eine anfänglich ausreichend hohe Ionenkonzentration innerhalb von wenigen Minuten um mehr als den Faktor 10 verringern kann. Dies verringert die Wirksamkeit der Luftionisierung und macht entsprechenden Gegenmaßnahmen erforderlich.

In JP 20063492989 A wird beispielsweise vorgeschlagen, einen flexiblen Luftleitschlauch einer Klimaanlage, die eine Ionisierungsvorrichtung aufweist, mit einer durchgängigen, elektrisch leitfähigen Beschichtung zu versehen. Die elektrisch leitfähige Beschichtung wird geerdet Im vorgeschlagenen Aufbau soll verhindert werden, dass die durch den Schlauch hindurch strömenden Ionen elektrisch neutralisiert werden, und damit verloren gehen.

In JP 2004-79471 A wird ein Verfahren beschirieben mit dem der Verlust bereits erzeugter Anionen in seinem, der Ionisierungsvorrichtung nachfolgendem. Luftleitkanal, verhindert werden soll. Dazu wird vorgeschlagen, dass die Ionisierungsvorrichtung nach einem durchgängigen Betrieb über eine Zeitdauer von 10 bis 40 Minuten hinweg für etwa 3 bis 7 Minuten ausgeschaltet wird. Im WO 2009/045438 A1 werden unterschiedliche organische Polymerzusammensetzungen, die mit einem metallischen Überzug versehen werden, für die Verwendung als Luftführungskanäle in Kraftfahrzeugen vorgeschlagen.

Das Dokument JP 2002277010 betrifft eine Klimaanlage mit einem Ionengenerstor und luftführenden Kanälen mit den Merkmalen des Oberbegriffs des Anspruchs 1. Ein Kanal der Klimaanlage weist einen Ventilator zum Einsaugen von atmosphärischer Luft und Weiterleiten der Luft in einen luftführenden Kanal, einen Filter zum Entfernen von Staub aus der Luft und einem negative Ionen erzeugenden Ionengenerator auf.

Das Dokument JP 2004079471 betrifft eine Klimaanlage mit einem Ionengenerator, der negative Ionen emittieren kann.

Das Dokument JP 2004182123 offenbart eine Klimaanlage für ein Kraftfahrzeug mit einer Ionenerzeugungseinrichtung mittels der Ionen erzeugt werden können. Die Ionenerzeugungseinrichtung ist in einem luftführenden Kanal der Klimaanlage angeordnet. Die Ionenerzeugungseinrichtung kann positive und negative Ionen in einem Clean-Modus erzeugen. In einem Refresh-Modus können negative Ionen erzeugt werden. Im Betrieb der Ionenerzeugungseinrichtung kann der Refresh-Modus dem Clean-Modus nachgeschaltet sein.

Das Dokument WO 2006/134681 A1 offenbart eine Luftführungsvorrichtung gemäß des Oberbegriffs des Anspruchs 1.

Obwohl die bereits vorgeschlagenen Maßnahmen grundsätzlich geeignet sind, das beschriebene Problem des verlusts von bereits erzeugten Ionen zu verringern, weisen die bereits vorgeschlagenen Verfahren und Vorrichtungen jedoch nach wie vor unterschiedlichste Probleme auf, wie beispielsweise hohe Kosten, nicht ausreichende Wirksamkeit und übermäßige elektrische Neutralisierung bereits erzeugter Ionen.

Es ist daher die Aufgabe der Erfindung eine Luftführungsvorrichtung vorzuschlagen, die besonders geeignet als Luftführungsvorrichtung für eine Ionisierungsvorrichtung ist und Vorzüge gegenüber bekannten Luftführungsvorrichtungen aufweiset. Eine weitere Aufgabe der Erfindung besteht darin eine gegenüber bekannten Ionisierungsvorrichtungen verbessert Ionisierungsvorrichtung vorzuschlagen.

Es wird daher vorgeschlagen, eine Luftführungsvorrichtung, insbesondere einen Luftführungskanal und/oder eine Luftausströmerdüse, bevorzugt eine Luftführungsvorrichtung für eine Ionisierungsvorrichtung (hier speziell einen Luftführungskanal und/oder eine Luftausströmerdüse für eine Ionisierungsvorrichtung) nach Anspruch 1 auszubilden.

Ionisierungsvorrichtungen sind in unterschiedlichen Bauausführungen bekannt. Beispielsweise können Ionisierungsvorrichtungen eine geeignet ausgebildete Elektrode aufweisen, an welche eine negative Hochspannung angelegt wird. Die Elektroden setzt dann negativ geladene Ionen frei, also sogenannte Anionen. Negative Ionen enthaltende Luft wird in allen Regel als "frische Luft" wahrgenommen. Dementsprechend wird üblicherweise auch von einer sogenannten "Relax-Betriebsart" (Relax für Entspannen) gesprochen. Zusätzlich oder alternativ ist es möglich, dass eine Ionisierungsvorrichtung auch eine geeignet geformte Elektrode aufweist, an welche eine positive Hochspannung angelegt wird. Die derart positiv geladene Höchspannungselektrode emittiert dementsprechend positiv geladene Ionen, sogenannte Kationen. Die erzeugten Kationen (zum Beispiel H⁺-Ionen) können mit anderen Molekülen und/oder Ionen kombinieren (zum Beispiel mit O₂⁻-Ionen zu HO₂⁻-Ionen), die ihrerseits reinigend (z.B. bakteriozid) wirken können. Deshalb wird hier üblicherweise von einem "Clean"-Betriebsmodus (Clean für sauber) gesprochen wird. Selbstverständlich ist es möglich, dass auch mehrere Elektroden (sowohl mit gleicher Hochspannungsversorgung, als auch mit unterschiedlicher Hochspannungsversorgung) vorgesehen werden. Typischerweise werden Ionisierungsvorrichtungen derart betrieben, dass ausschließlich oder zumindest überwiegen Ionen eines bestimmten Ionentyps - also beispielsweise Anionen, wie insbesondere O₂⁻-Ionen und/oder HO₂⁻-Ionen - erzeugt werden (selbst dann, wenn die Ionisierungsvorrichtung über unterschiedlich gestaltete Elektroden verfügt). Dadurch kommt es in aller Regel zu einer elektrostatischen Aufladung von Bauteilen (wie insbesondere von Luftführungsvorrichtungen wie beispielsweise Luftführungskanälen und/oder Luftausströmerdüsen), die in Luftströmungsrichtung gesehen der Ionisierungsvorrichtung (Insbesondere dem eigentlichen Ionisierungsmittel) nachgeschaltet sind. Aufgrund der im Luftstrom enthaltenen Ionen werden die entsprechenden Bauteiloberflächen elektrostatisch geladen. Dadurch entstehen elektrische Felder, welche wiederum einen Einfluss auf die Luftströmung, insbesondere auf die in der Luftströmung enthaltenen Ionen, haben. Somit entsteht ein hochkomplexes, auch numerisch kaum beschreibbares dynamisches Gebilde. Die unterschiedlichen dynamischen Ladungsverteilungen bewirken jedoch in aller Regel eine "Behinderung" der im Luftstrom mitgeführten Ionen (meist dadurch, dass die Ionen abgelenkt werden und anschließend durch einen Wandkontakt vernichtet werden). Experimente haben ergeben, dass sich bereits nach wenigen Minuten eine drastische Verringerung der schlussendlich in einen Kraftfahrzeugihnenraum freigesetztten Ionenkonzentration ergeben kann. Die Zeitspanne, nach der es zu einer signifikanten Einbuße an freigesetzten Luftionen (bis zu einem Faktor 10 und mehr) kommen kann, ist dabei überraschenderweise deutlich kürzer als bislang angekommen. Versuche haben ergeben, dass die Reduktion der freigegebenen Ionen um einen Faktor 10 bereits nach 1 bis 5 Minuten erreicht wird. Von daher ist es auch bei typischen Betriebszyklen bei einem Kraftfahrzeug (beispielsweise bei einem Automobil) erforderlich, entsprechende Gegenmaßnahmen vorzugehen, die der beschriebenen Verringerung der Anzahl der in den Kraftfahrzeuginnenraum freigesetzten Ionen entgegen wirkt. Vorliegend wird vorgeschlagen zu diesem Zweck zumindest bereichsweise zumindest ein lokal wirkendes Feldkompensationsmittel vorzusehen. Mit Hilfe des vorgeschlagenen, zumindest einen Iokalen Feldkompensationsmittel ist es insbesondere möglich, zumindest zeitweise und/oder zumindest bereichsweise eine lokale Feldkompensation von elektrischen Feldern mit Hilfe von Spiegelladungen zu ermöglichen. Besonders vorteilhaft ist es dabei, wenn das beziehungsweise die lokal wirkenden Feldkompensationsmittel als passiv wirkende lokal wirkende Feldkompensationsmittel ausgebildet sind, sodass beispielsweise keine aufwändige Steuerelektronik erforderlich ist und/oder Energie zum Betrieb des Feldkompensationsmittels (beziehungsweise der gegebenenfalls vorhandenen mehreren Feldkompensationsmittel) bereit gestellt werden muss. Die Erfinder haben zu ihrer eigenen Überraschung herausgefunden, dass nicht etwa besonders gut leitende, flächig aufgetragene Materialien und/oder besonders gut isolierende Oberflächen eine besonders hohe Konzentration von schlussendilch freigesetzten Ionen (beispielsweise in einem Kraftfahrzeuginnenraum hinein) bewirkt. Vielmehr hat es sich gezeigt, dass eine besonders hoher Ionenaustrag dann erfolgen kann, wenn die der Ionisierungsvorrichtung nachgeschalteten Bauteile (speziell Bauteile des beziehungsweise der lokal wirkenden Feldkompensationsmittel) über größere Entfernungen hinweg eine relativ niedrige elektrische Leifähigkeit aufweisen und/oder in einem kleinräumigen Bereich eine relativ hohe elektrische Leitfähigkeit aufweisen. Dadurch kann eine nur lokal wirkende elektrische Feldkompensation realisiert werden, wohingegen im großräumigen Bereich keine elektrische Feldkompensation (bzw. nur eine geringe bzw. langsam erfolgende - und damit in aller Regel "hinterher hinkende" elektrische Feldkompensation) erfolgen kann. Großräumige und elektrisch gut leitfähige Beschichtungen dagegen können inhomogene Aufladungen nur ausmitteln, was einer nur lokal wirkenden elektrischen Feldkompensation entgegensteht. Die Größenordnung der lokalen Strukturen liegt dabei typischerweise in einem Bereich von wenigen Zentimetern (bzw. Quadratzentimetern), beispielsweise mit typischen Längen und/oder Breiten von 0,5 cm, 1 cm, 1,5 cm, 2 cm, 3 cm, 4 cm oder 5 cm. Dadurch, dass mit Hilfe einer Luftführungsvorrichtung entsprechend der vorgeschlagenen Ausbildung ein besonders großer Anteil an Ionen durch die Luftführungsvorrichtung hindurch geführt werden kann, ohne dass diese vernichtet werden, ist es möglich die Ionisierungsvorrichtung beispielsweise kleiner und kompakter auszubilden und gegebenenfalls auch den erforderlichen Energieverbrauch der Ionisierungsvorrichtung zu reduzieren. Dadurch ist es wiederum möglich, eine besonders effektive Gesamtanlage zu realisieren.

Es hat sich als besonders vorteilhaft erwiesen, wenn zumindest ein lokal wirkendes Feldkompensationsmittel zumindest bereichsweise als eine Mehrzahl von elektrisch leitfähigen Materialabschnitten ausgebildet ist. Ein derartiges, lokal wirkendes Feldkompensationsmittel weist eine nur geringe Tendenz zur Vernichtung von Ionen, bei gleichzeitig relativ einfachem und kostengünstigem Aufbau auf. Die Größendimensionierungen für die vorgeschlagene Ausbildung der elektrisch leitfähigen Materialabschnitte (insbesondere hinsichtlich deren Länge und/oder Breite) liegt üblicherweise in einem Bereich von bis zu 0,5 cm, 1 cm, 1,5 cm, 2 cm, 3 cm, 4 cm oder 5 cm.

Vorteilhaft ist es, wenn die elektrische Leitfähigkeit zumindest eines elektrische leitfähigen Materialabschnitts größer als oder gleich 10⁴ S/m, 10⁵ S/m und/oder 10⁶ S/m ist (S für Siemens und m für Meter). Derartige elektrische Leitfähigkeiten haben sich bewährt, um eine besonders gute, lokale FeldKompensation realisieren zu können.

Besonders vorteilhaft ist es, wenn bei der Luftführungsvorrichtung zumindest zwei lokal wirkende Feldkompensationsmittel, insbesondere zumindest zwei elektrisch leitfähige Matertalabschnitte voneinander elektrisch entkoppelt sind. Auf diese Weise ist es besonders einfach, die Feldkompensation auf lokale Effekte zur begrenzen. Dadurch kann die Effektivität der vorgeschlagenen Luftführungsvorrichtung erhöht werden. Die elektrische Entkupplung kann dabei im einfachsten Fall durch eine Materialunterbrechung des elektrisch leitfähigen Materials erfolgen, insbesondere dann, wenn das Trägermaterial (beispielsweise das Material der Luftführungsvorrichtung) eine relativ geringe Leitfähigkeit aufweiset.

Als vorteilhaft hat es sich erwiesen, wenn die elektrische Entkopplung der zumindest zwei lokal wirkenden Feldkompensationsmittel zumindest teilweise eine Leitfähigkeit von weniger ale oder gleich 10⁵ S/m, 10⁴ S/m, 10³ S/m, 10² S/m, 10¹ S/m und/oder 10⁰ S/m aufweist. Die genannten, relativ niedrigen elektrische Leitfähigkeiten haben sich für die vorliegend vorgeschlagene Luftführungsvorrichtung als besonders geeignet erwiesen.

Zusätlich oder alternativ ist es auch möglich, dass sogenannte ESD-Folien oder dergleichen Bauteile verwendet werden (ESD für Electro Static Device). Auch hier hat es sich gezeigt, dass derartige Bauteile im besonderen Maße als lokales Feldkompensationsmittel geeignet sind und dementsprechend ein besonders hoher Anteil der erzeugten Ionen von den entsprechenden Bauteilen "durchgelassen" wird. Dennoch kann der Aufbau der entsprechenden Ionisierungsvorrichtung nach wie vor relativ einfach und kostengünstig sein. Insbesondere ist darauf hinzuweisen, dass sich die Montage der entsprechenden Bauteile (wie beispielsweise der ESD-Folien) besonders einfach gestauten kann, da diese als einzelne, große Flächen angebracht werden können. Dies kann die Herstellung der Luftführungsvorrichtung nochmals vereinfachen. Bevorzugt aber nicht zwingend kann der so behandelte oder mit dem elektrisch geringfügig leitenden Bauteil ausgerüstete Luftführungskanal durch eine leitfähige Verbindung mit einem Massepotential verbunden sein, um eine entstehende Aufladung gegebenenfalls abführen zu können.

Es hat sich als besonders vorteilhaft erwiesen, wenn zumindest ein elektrisch geringfügig leitendes Bauteil zumindest bereichsweise eine elektrische Leitfähigkeit von kleiner als oder gleich 10⁵ S/m, 10⁴ S/m, 10³ S/m, 10² S/m, 10¹ S/m, 10⁰ S/m, 10⁻¹ S/m, 10⁻² S/m, 10⁻³ S/m, 10⁻⁴ S/m oder 10⁻⁵ S/m aufweist Die Verwendung von Materialien mit den genannten Leitfähigkeiten hat sich in ersten Versuchen als besonders geeignet erwiesen.

Erfindungsgemäß ist das lokal wirkendes Feldkompensationsmittel, insbesondere zumindest ein elektrisch leitfähiger Material-abschnitt und/oder zumindest eine elektrisch geringfügig leitendes Bauteil flächig und folienartig ausgebildet. Erste Versuche haben ergeben, dass es bei den lokal wirkenden Feldkompensationsmitteln in aller Regel nicht (oder nur in geringfügigem Ausmaß) auf deren Tiefe (insbesondere deren Bauteilabmessungen in einer senkrecht zur Luftströmungsrichtung stehenden Richtung) ankommt. Mit einem flächigen bzw. folienartigen Aufbau kann daher eine hochwirksame Vorrichtung bei geringem Materialverbrauch (und damit niedrigem Gewicht und/oder geringen Kosten) ausgebildet werden. Typische Dicken von flächig ausgebildeten Bauteilen und/oder Folien liegen im Bereich von 0,1 mm.

Eine weitere besonders bevorzugte Ausführung der Luftführungsvorrichtung ergibt sich darüber hinaus, wenn zumindest ein lokal wirkendes Feldkompensationsmittel, insbesondere zumindest ein elektrisch leitfähiger Materialabschnitt und/oder zumindest ein elektrisch geringfügig leitendes Bauteil selbstklebend ausgeführt ist. Bei einer derartigen Bauausführung kann eine besonders einfache Montage der lokal wirkenden Feldkompensationsmittel an der Luftführungsvorrichtung (wie beispielsweise an einem Luftführungskanal, an einer Luftausströmerdüse, gegebenenfalls auch an sonstigen Baugruppen) realisiert werden. Darüber hinaus ist darauf hinzuweisen, dass Metallfolien und/oder ESD-Folien bereits zum jetzigen Zeitpunkt oftmals in selbstklebender Form handelsüblich in großen Mengen erhältlich sind. Dadurch kann eine nochmalige Kostenreduktion erzielt werden und eine schnelle Umsetzung der vorliegend vorgeschlagenen Luftführungsvorrichtung kann besonders rasch erfolgen.

Weiterhin wird eine Ionisierungsvorrichtung vorgeschlagen, die zumindest ein Ionisierungsmittel zur zumindest zeitweisen Freisetzung von Ionen eines ersten Ionentyps, sowie zumindest eine Luftführungsvorrichtung mit dem vorab beschriebenen Aufbau aufweist. Eine derart ausgeführte Ionisierungsvorrichtung kann es möglich machen, dass ein besonders hoher Anteil der von der Ionisierungsvorrichtung erzeugen Ionen am eigentlichen "Ziel" ankommt (also insbesondere in einen Kraftfahrzeuginnenraum hinein freigesetzt wird). Dadurch kann die Gesamtionisierungsvorrichtung besonders einfach, kostengünstig und effektiv aufgebaut Werden. Da beispielsweise ein besonders hoher Anteil der von der Ionisierungsvorrichtung erzeugten Ionen "überlebt", kann eine bestimmte Ionenkonzentration am eigentlichen Luftauslass auch dann erzielt werden, wenn eine im Verhältnis zu bekannten Ionisierungsvorrichtungen nur geringe Ionenkonzentration am Ort des Ionisierungsmittels erzeugt wird. Dadurch kann das Ionisierungsmittel Insbesondere kleiner, weniger leistungsfähig, leichter und energleverbrauchsärmer ausgebildet werden.

Die Effektivität der vorliegend vorgeschlagenen Ionisierungsvorrichtung kann nochmals deutlich gesteigert werden, wenn diese derart ausgebildet und eingerichtet ist, dass sie zumindest zeitweise in zumindest einem Regenerationsmodus betrieben werden kann. Während der Regenerationsphasen kann durchaus bewusst in Kauf genommen werden, dass sich über einen gewissen - möglichst kurzen - Zeitraum hinweg das (eigentlich) gewünsche Konzentrationsgemisch und/oder die Konzentration der in den Kraftfahrzeuginnenraum freigesetzten Ionen ändert. Es ist sogar denkbar, dass kurzzeitig die Anzahl der freigesetzten Ionen (zumindest bei einigen Ionentypen) auf 0 zurückgefahren wird. Dennoch ist es trotz (beziehungsweise dank) des Einfügens derartiger Regenerationsphasen möglich, die im zeitlichen Mittel freigesetzten Menge an Ionen zu erhöhen bzw. das Konzentrationsverhältnis der in den Kraftfahrzeuginnenraum freigesetzten Ionen dem gewünschen Zielwert besonders nahe kommen zu lassen. Insbesondere ist dies dadurch möglich, dass während der "Aktiv"-phasen, die zwischen den Regeneratonsphasen liegen, beispielsweise eine vergleichsweise hohe Konzentration an Ionen in den Kraftfahrzeuginnenraum abgegeben werden kann. Mit der vorgeschlagenen Weiterbildung ist es insbesondere möglich, die Effektivität der Ionisierungsvorrichtung nochmals gegebenenfalls deutlich zu erhöhen. In der Regel wirkt die Regenerationsphase dabei weniger auf das eigentliche Ionisierungsmittel, als vielmehr auf die dem Ionisierungsmittel im Luftstrom nachgesehalteten Bauteile (also insbesondere auf im Luftstrom nachgeschaltete Luftführungsvorrichtungen, wie beispielsweise Luftführungskanäle und/oder Luftauslassdüsen).

Die Ionisierungsvorrichtung kann insbesondere derart betrieben werden, dass in zumindest einem Regenerationsmodus zumindest zeitweise Ionen von einem zweiten, vom ersten Ionentyps unterschiedlichen Ionentyp freiesetzt werden und/oder zumindest zeitweise keine Ionen, insbesondere keine Ionen vom ersten Ionentyp freigesetzt wurden. Unter einem Ionentyp kann ein unterschiedliches Vorzeichen der Ladung zu versehen sein. Wenn also beispielsweise die Ionen des ersten Ionentyps Anionen sind, so kann es sich dementsprechend bei den Ionen vom zweiten Ionentyp um Kationen handeln (die gegebenenfalls auch nur in Form einer "Zwischenstufe" temporär vornegen könnten). Zusätzlich oder alternativ können die Ionen jedoch auch eine unterschiedliche Ladung (beispielsweise einfache, zweifache, dreifache usw. Elementarladung) aufweisen. Insbesondere kann es sich zusätzlich oder alternativ auch um unterschiedliche Moleküle handeln, die ionisiert werden beziehungsweise ionisiert wurden (wie beispielsweise die bereits vorab beschriebenen Ionen O₂⁻ und HO₂⁻). Insbesondere dann, wenn Ionen mit entgegengesetzter Ladung während des Regenerationsmodus freigesetzt werden, können die gegebenenfalls bereits elektrostatisch geladenen Bereiche der dem eigentlichen Ionisierungsmittel nachgeschalteten Bauteile durch die Platzierung entgegengesetzter Ladungsträger entladen werden. Auch ist es möglich, quasi eine entgegengesetzte Vorab-Ladungsverteilung zu erzeugen, so dass in einer ersten, der Regenerationsphaae nachgeschalteten Betriebsphase der Ionisierungsvorrichtung zunächst die dem eigentlichen Ionisierungsmittel nachgeschalteten Bauteile entladen werden, und erst anschließend durch die Ionen des ersten Ionentyps neu aufgeladen werden. Insbesondere dann, wenn eine Regeneration mit Hilfe von Ionen eines zweiten Ionentyps erfolgt, kann die Dauer der Regenerationsphase typischerweise in einem Bereich zwischen 5 und 30 Sekunden liegen, während die "normale" Betriebsdauer in einem Intervall zwischen 30 Sekunden und 3 Minuten liegt. Die Ionen vom zweiten Ionentyp können dabei zumindest zeitweise zusätzlich und/oder zumindest zeitweise alternativ zu den Ionen des ersten Ionentyps freigesetzt werden. Es ist also möglich, dass (zumindest zeitweise) während der Regenerationsphase vorwiegend oder (im Wesentlichen) ausschließlich Ionen vom zweiten Ionentyp freigesetzt werden. In diesem Fall kann die Dauer der Regenerationsphase typischerweise besonders kurz gehalten werden. Ebenso ist es jedoch auch möglich, dass die Ionen vom zweiten Ionentyp freigesetzt werden, während die Erzeugung von Ionen vom ersten Ionentyp im Wesentlichen kontinuierlich fortgeführt wird. Hierdurch kann eine höhere Konstanz an Freisetzung von "frischer Luft" in den Fahrzeuginnenraum hinein erreicht werden. Darüber hinaus kann in der Regel die Ansteuerung der entsprechenden Elektrode einfacher gestaltet werden. Gemäß dem obigen Vorschlag ist es auch möglich, dass bei zumindest einem Regenerationsmodus zumindest zeitweise keine Ionen, insbesondere zumindest zeitweise keine Ionen vom ersten Ionentyp freigesetzt werden. Dies kann beispielsweise durch ein vollständiges Ausschaben der Ionisierungsvorrichtung realisiert werden. Auch hat es sich gezeigt, dass bereits nach relativ kurzen Zeitdauern eine Regeneration der Gesamtanordnung erfolgen kann. Typischerweise reichen Ausschaltzeiten in einem Bereich zwischen 5 und 30 Sekunden aus (bei Einschaltzeiten im normalen Betriebsmodus von typischerweise 30 Sekunden bis 3 Minuten). Diese Lösung ist insbesondere deshalb von Vorteil, weil sie konstruktiv besonders einfach realisiert werden kann. Insbesondere kann dieses Verfahren für Ionisierungsvorrichtungen verwendet werden, die lediglich eine einzelne Hochspannungsquelle und/oder eine einzelne Hochspannungselektrode aufweisen. Eine besonders schnelle Regeneration kann dabei insbesondere dann realisiert werden, wenn relativ feuchte Luft vorliegt, da diese eine schnelle Entladung elektrostatischer Ansammlungen begünstigt.

Die Ansteuerung der Ionisierungsvorrichtung kann dabei durch zumindest eine Steuervorrichtung zur Ansteuerung der Ionisierungsvorrichtung erledigt werden. Als derartige Steuervorrichtung kann beispielsweise ein Einplatinen. Computer oder dergleichen dienen. Möglich ist es dabei, dass eine spezielle Steuervorrichtung zur Ansteuerung des Regenerationsmodus vorgesehen wird. Ebenso ist es aber auch möglich, dass die Funktionalität von einer bei Kraftfahrzeugen typischerweise ohnehin vorhandenen elektronischen Steuerung (beispielsweise eine elektronische Steuerung für eine Kraftfahrzeugklimaanlage) mit übernommen wird. Da die Rechenlast für eine Steuervorrichtung typischerweise relativ klein ist, stellt diese zusätzliche Arbeitslast in aller Regel kein Problem dar.

Weiterhin wird eine Klimaanlage, insbesondere eine Kraftfahrzeugklimaanlage vorgeschlagen, welche zumindest eine Luftführungsvorrichtung mit dem oben beschriebenen Aufbau und/oder zumindest eine Ionisierungsvorrichtung mit dem vorab beschriebenen Aufbau aufweist. Die Klimaanlage weist dann die bereits im Zusammenhang mit der vorab beschriebenen Luftführungsvorrichtung und/oder mit der vorab beschriebenen Ionisierungsvorrichtung genannten Eigenschaften und Vorteile in analoger Weise auf. Bei dem Kraftfahrzeug, für das die Kraftfahrzeugklimaanlage bestimmt ist, kann es sich in beliebiger Weise um ein Wasserfahrzeug, ein Luftfahrzeug und/oder ein Landfahrzeug (schienengebunden/nicht schlenengebunden) handeln.

Im Folgenden wird die Erfindung anhand vorteilhafter Ausführungsbeispiele und unter Bezugnahme auf die beigefügte Zeichnung näher erläutert. Es zeigt:
- Fig. 1:: eine Kraftfahrzeugklimaanlage mit einer Ionieierungsvorrichtung im schematischen Querschnitt;
- Fig. 2:: der zeitliche Verlauf der von einer üblichen Kraftfahrzeugkilmaanlage in den Kraftfahrzeuginnenraum ausgegebenen Ionen-Konzentration in einem Dauerbetriebsverfahren;
- Fig. 3:: der zeitliche Verlauf der von einer Kraftfahrzeugklimaanlage in den Kraftfahrzeuginnenraum ausgegebenen Ionenkonzentration unter Verwendung eines ersten Typs von Regenerationsverfahren;
- Fig. 4:: der zeitliche Verlauf der von einer Kraftfahrzeugklimaanlage in den Kraftfahrzeuginnenraum ausgegebenen Ionenkonzentration unter Verwendung eines zweiten Typs von Regenerationsverfahren;
- Fig. 5:: ein erstes Ausführungsbeispiel für einen Luftleitkanal mit lokal wirkendem Feldkompensationsmittel in schematischer, perspektivischer Ansicht;
- Fig. 6:: ein zweites Ausführungsbeispiel für einen Luftführungskanal mit lokal wirkendem Feldkompensationsmittel in schematischer, perspektivischer Ansicht.

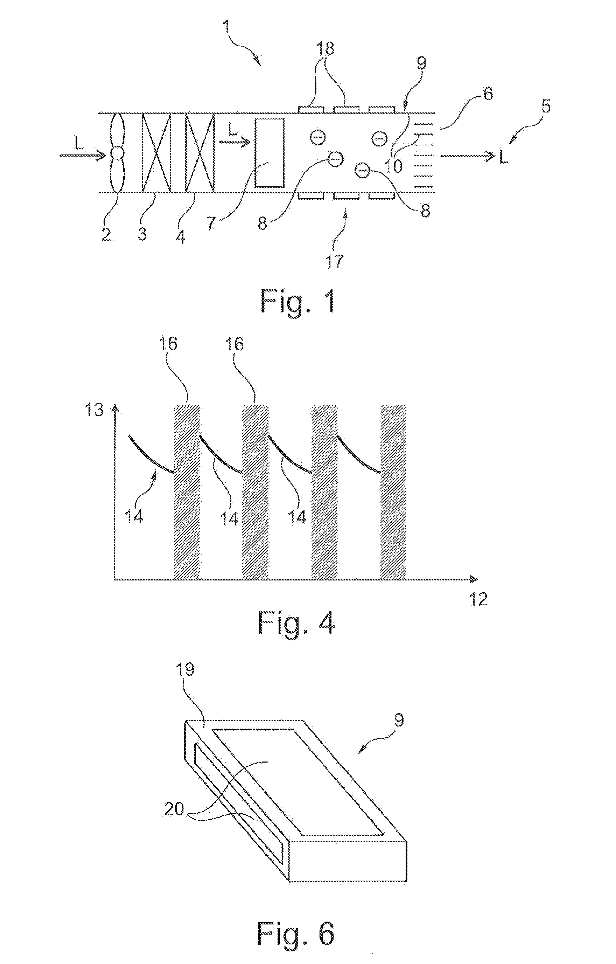

In Fig. 1 ist eine Kraftfahrzeugklimaanlage in einer schematischen Querschnittsansicht dargestellt. Die Kraftfahrzeugkilmaanlage 1 ist dabei in einer stark vereinfachenden Ansicht dargestellt, die sich im Wesentlichen auf die Bereiche konzentriert, die im Zusammenhang mit der Luftaufbereitung durch Ionisierung der von der Kraftfahrzeugklimaanlage 1 aufzuarbeitenden Luftströmung L stehen.

Die von der Kraftfahrzeugklimaanlage 1 mit Hilfe eines Gebläses 2 angesaugte Luft L wird zunächst durch einen Filter 3 geleitet, in dem Pollen, Schmutzpartikel und dergleichen die in der Umgebungsluft enthalten sein können, ausgefiltert wurden. Anschließend wird die Luftströmung L durch einen Heizkörper 4 geleitet und entsprechend temperiert (selbstverständlich kann die Temperierung auch durch eine Mischung von Warm- und Kaltluft erfolgen; darüber hinaus kann die Luft auch mit Hilfe eines Verdampfers gekühlt werden usw.). Die bereits weitgehend aufbereitete Luftströmung L wird, bevor sie über eine Luftausströmerdüse 6 in einen Kraftfährzeuginnenraum 5 freigegeben wird, zunächst durch ein Ionisierungsmodul 7 geleitet. In diesem Ionisierungsmodul 7 werden beispielsweise Anionen 8 erzeugt, die von den Kraftfahrzeuginsassen als "frische Luft" wahrgenommen werden. Die vom Ionisierungsmodul 7 erzeugten Anionen 8 müssen dabei zunächst über entsprechend geformte Luftleitkanäle 9 hindurch geführt werden sowie durch die Luftausströmerdüse 6 hindurch strömen. Dabei treffen einige der vom Ionisierungsmodul 7 erzeugten Anionen 8 auf die Wände des Luftleitkanals 9 bzw. der Luftausströmerdüse 6 auf und geben ihre Ladung an den entsprechenden Randbereich 10 ab. Mit der Zeit kommt es dadurch zu einer elektrostatischen Aufladung bestimmter Wandbereiche 10 von Luftleitelement 9 und Luftausströmerdüse 6. Die elektrostatische Aufladung der entsprechenden Wandbereiche 10 ist jedoch nicht gleichmaßig, sodass zwischen unterschiedlich stark geladenen Wandbereichen 10 elektrische Felder auftreten. Die elektrischen Felder beeinflussen Wiederum die Luftdurchströmung L (Insbesondere die in der Luftdurchströmung L enthaltenden Ionen), insbesondere die Bewegungsbahn der Anionen 8, sodass sich das System überaus dynamisch und nur schwer vorhersagbar verhält.

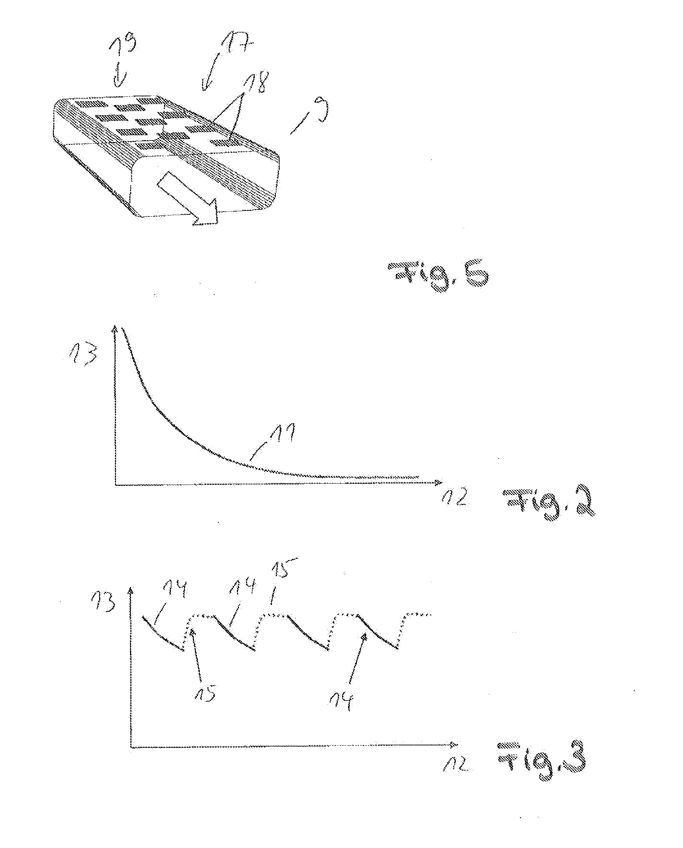

In aller Regel bewirkt die elektrostatische Aufladung der Wandbereiche 10 von Luftleitkanal 9 und Luftausströmerdüsen 6 jedoch eine starke Reduktion der in den Kraftfahrzeuginnenraum 5 schlussendlich austretenden Anionen 8, sofern nicht geeignete Gegenmaßnahmen ergriffen werden. Dementsprechend müsste das Ionisierungsmodul 7 eine entsprechend größere Anzahl an Anionen 8 erzeugen. Dafür müsste das Ionisierungsmodul 7 entsprechend größer ausgelegt werden (wodurch es teurer und schwerer würde), und entsprechend mehr elektrische Energie müsste für das Ionisierungsmodul 7 bereit gestellt werden. Die Abnahme der Konzentration der in den Kraftfahrzeuginnenraum 5 freigegebenen Anionen 8 ist in Fig. 2 schematisch als Funktionsgraf 11 dargestellt (sofern keine gesonderten Maßnahmen, wie beispielsweise besondere Betriebsverfahren und/oder konstruktive Maßnahmen ergriffen werden). In Fig. 2 ist entlang der Abszisse 12 die Zeit t dargestellt, wohingegen entlang der Ordinate 13 die Ionenkonzentration, die in den Kraftfahrzeuginnenraum 5 freigegeben wird, ausgetragen ist. Deutlich erkennt man die starke Abnahme der Ionenkonzentration. Typischerweise erreicht nach einer Zeitdauer von 1 bis 5 Minuten nur noch jedes zehnte erzeugte Anlon 8 den Kraftfahrzeuginnenraum 5. Der Effekt ist also überaus signifikant.

Um die im zeitlichen Mittel in den Kraftfahrzeuginnenraum 5 freigegebene Konzentration an Anionen 8 zu erhöhen, wird vorgeschlagen, konstruktive Maßnahmen vorzusehen, die aufgrund ihrer Eigenschaften den Anteil der in den Kraftfahrzeuginnenraum 5 freigegebene Anionen 8 (oder sonstiger Ionen) gegenüber Standard-Kraftfahrzeugklimaanlagen erhöhen.

Eine Möglichkeit für eine derartige konstruktive Maßnahme besteht beispielsweise in der bereits in Fig. 1 angedeuteten Anordnung von einem Raster 17 von jeweils zueinander beabstandet angeordneter Metallfolien 18. Mögliche Details zur Lage und Anordnung der Metallfolien 18 am Luftleitkanal 9 sind darüber hinaus in Fig. 5 zu erkennen. Wie man erkennen kann, wird beim vorliegend dargestellten Ausführungsbeispiel ein regelmäßiges Raster aus gleichartig geformten Metallfolien 18 verwendet. Die einzelnen "Spalten" des Rasters sind dabei jeweils zueinander versetzt, so dass die Matallfolien 18 gewissermaßen "auf Lücke" gegeneinander angeordnet sind. Die Metallfolien 18 sind als selbstklebende Metallfolien ausgebildet, und werden beispielsweise auf den bereits fertig ausgebildeten Luftleitkanal 9 (der beispielsweise mit Hilfe eines Spritzgussverfahrens aus Kunststoff gefertigt wurde) aufgeklebt. Wie aus den Fig. 1 und 5 zu erkennen ist, befinden sich die Metallfolien 18 dabei auf der Außenseite 19 des Luftleitkanals 9.

Im vorliegend dargestellten Ausführungsbeispiel sind die Metallfolien 18 jeweils voneinander elektrisch isoliert angeordnet und darüber hinaus nicht geerdet. In einem weiteren denkbaren Ausführungsbeispiel ist es auch möglich, dass die (beziehungsweise ein Teil der) Metallfolien 18 elektrisch miteinander verbunden sind (gegebenenfalls Ober hochohmige elektrische Leiter) und/oder (gegebenenfalls über einen hochohmigen elektrischen Leiter) an Erdpotential gelegt sind.

Unabhängig von der konkreten Detailausführung hat sich gezeigt, dass die lokal begrenzte Beweglichkeit von Ladungsträgern in den einzelnen Metallfolien 18 bewirkt, dass über eine begrenzte Wegstrecke hinweg in den Metallfolien 18 Spiegelladungen entstehen können, die zu elektrischen Landungen bzw. Ladungsclustem an den inneren Wandbereichen 10 und/oder im Inneren des Luftleitkanals 9 korrespondieren können. Die dadurch gebildeten Spiegelladungen bewirken eine Reduktion bzw. vorteilhafte Umverteilung der vorhandenen elektrischen Felder, was schlussendlich zu einem höheren Anteil durch den Luftleitkanal 9 hindurch tretender Anionen 8 führen kann (ohne dass diese an den Wandbereichen 10 des Luftleitkanals 9 bzw. des Luftausströmerelements 6 verloren gehen). Dadurch kann der Austritt von Anionen 8 in den Kraftfahrzeuginnenraum 5 verbessert werden.

Ein weiteres mögliches Ausführungsbeispiel für eine Vorrichtung, die eine derartige, lokal wirkende Feldkompensation bewirkt, ist in Fig. 6 vorgestellt. Hier ist ebenfalls ein Luftleitkanal 9 in einer schematischen perspektivischen Ansicht dargestellt. Auf den Außenseiten 19 des Luftleitkanals 9 sind dabei großflächig angebrachte, sogenannte ESD-Folien 20 vorgesehen. Die ESD-Folien 20 sind im vorliegend dargestellten Ausführungsbeispiel als selbstklebande Folien ausgebildet, die auf den fertig ausgebildeten Luftleiftkanal 9 aufgeklebt werden. Aufgrund der in Verhältnis zu Metallfolien schlechten elektrischen Leitfähigkeit - im Verhältnis zu elektrischen Isolatoren jedoch guten elektrischen Leitfähigkeit - resultiert auch hier effektiv eine lokale Kompensation auftretender elektrischer Felder, wobei diese Kompensation nur In einem relativ eng begrenzten Flächenbereich auftritt. Es scheint sich so zu verhalten, dass über relativ kurze räumliche Distanzen hinweg (beispielsweise einige wenige Zentimeter) der elektrische Widerstand der ESD-Folie 20 für die Ausbildung von Spiegelladungen nicht negativ hervortritt. Über längere räumliche Entfernungen hinweg (beispielsweise 10 Zentimeter oder mehr), scheint der elektrische Widerstand der ESD-Folien 20 dagegen effektiv eine Behinderung für die Bewegung elektrischer Spiegelladungen, und damit eine Behinderung der Kompensation der elektrischen Felder darzustellen. Eine weitere Erklärungsmöglichkeit für die Eigenschaften der ESD-Folie 20 besteht darin, dass die ESD-Folle 20 nur eine geringe Längsleitfähigkeit aufweist. Diese nur geringe Längsleitfähigkeit lässt, eine lokal stark variierende Bildladung (auch über kurze Distanzen hinweg) zu, beziehungsweise macht die nur geringe Längsleitfählgkeit es möglich, dass lokal stark unterschiedliche Oberflächenladungen abgeführt werden können. Auch im vorliegend dargestellten Ausführungsbeispiel von Fig. 6 ist es möglich, dass die einzelnen ESD-Folien 20 (gegebenenfalls hochohmig) elektrisch miteinander verbunden werden und/oder (gegebenenfalls über hochohmige Ver-bindungsleitungen) mit Masse verbunden werden.

Obwohl mit den vorgeschlagenen konstruktiven Maßnahmen (also beispielsweise dem Aufbringen von Metallfolien 18 und/oder ESD-Folien 20; vergleiche mit Fig. 5 bzw. Fig. 6) bereits eine gegenüber Standard-Kraftfahrzeugklimaanlagen deutlich erhöhte Ionenkonzentration in den Kraftfahrzeuginnenraum 5 freigegeben werden kann, empfiehlt es sich, zusätzlich zu den vorgeschlagenen konstruktiven Maßnahmen (bzw. gegebenenfalls zusätzlich zu sonstigen denkbaren konstruktiven Maßnahmen) eine besondere Art der Ansteuerung der Kraftfahrzeugklimaanlage 1 vorzusehen, um die im zeitlichen Mittel in den Kraftfahrzeuginnenraum 5 abgegebene Konzentration von Anionen 8 weiter zu erhöhen.

Um die im zeitlichen Mittel In den Kraftfahrzeuginnenraum 5 freigegebene Konzentration an Anionen 8 zu erhöhen, ist es beispielsweise möglich, das Ionisierungsmodul 7 nur über einen gewissen Zeitraum (typischerweise 1 bis 3 Minuten) hinweg in einem normalen Betriebsmodus 14 zu betreiben (vergleiche mit Fig. 3). Nach Ablauf einer gewissen Zeitspanne wird die Kraftfahrzeugklimaanlage 1 in einen Regenerationsmodus 15 geschaltet, in dem nicht nur Anionen 8 (wie in Fig. 1 dargestellt) erzeugt werden, sondern in dem zusätzlich auch Kationen von dem Ionisierungsmodul 7 (was hiefür entsprechend ausgestaltet sein muss) erzeugt werden. Die vom Ionisierungsmodul 7 erzeugten Kationen werden im vorliegend dargestellten Ausführungsbeispiel nur "temporär" erzeugt und dienen lediglich zur Erzeugung eines zweiten Ionentyps, wobei es sich vorliegend ebenfalls um Anionen 8 (wenn auch von einem unterschiedlichen Ionentyp) handelt. Durch die "wechselweise" Erzeugung von unterschiedlichen Ionentypen kommt es zu einer Entladung der statisch geladenen Wandbereiche 10, sodass dank der Regenerationsphase 15 die Konzentration der in den Kraftfahrzeuginnenraum 5 freigegebenen Anionen 8 nicht nur nicht weiter abnimmt, sondern im Gegenteil wieder zunehmen kann, Nach Ablauf der Regenerationsphase 15 (welche typischerweise 5 bis 30 Sekunden andauert) schließt sich erneut ein normaler Betriebsmodus 14 an, welcher wiederum von einem Regenerationsmodus 15 gefolgt wird usw.. Dieses Verfahren ist in Fig. 3 dargestellt. Auch hier ist längs der Abszisse 12 die Zeit dargestellt, wohingegen längs der Ordinate 13 die Konzentration der in den Kraftfahrzeuginnenraum 5 freigesetzten Anionen dargestellt ist.

Ein zweites mögliches Verfahren zur Ansteuerung eines Ionisierungsmoduls 7 besteht dann, dass das Ionisierungsmodul 7 - analog zum vorigen Ausführungsbeispiel - zunächst in einem normalen Betriebsmodus 14 betrieben wird (vergleiche mit Fig. 4). Nach Ablauf einer gewissen Zeitdauer (typischerweise 1 bis 3 Minuten) wird das Ionisierungemodul 7 dadurch regeneriert 16, dass das Ionisierungsmodul 7 einfach ausgeschaltet wird, und somit keinerlei Ionen (insbesondere keine Anionen 8) erzeugt werden. In dieser Regenerationsphase 16 können sich ebenfalls die statischen Aufladungen entlang der Wandbereiche 10 des Luftleitkanals 9 sowie der Luftausströmerdüse 6 abbauen, beispielsweise durch die üblicherweise im Luftstrom L befindliche Feuchtigkeit. Die Dauer der Regenerationsphase beträgt typischerweise 5 bis 30 Sekunden. Nach Ablauf der Regenerationsphase 16 erfolgt erneut ein Zyklus, in dem das Ionisierungsmodul 7 erneut in einem normalen Betriebsmodus 14 betrieben wird, anschließend erneut durch Ausschalten degeneriert 16 wird usw. Das beschriebene Verfahren ist in Fig. 4 näher illustriert. Auch hier ist entlang der Abszisse 12 die Zeit aufgetragen, wohingegen längs der Ordinate 13 die in den Kraftfahrzeuginnenraum 5 freigegebene Konzentration an Anionen 8 dargestellt ist.

Weitere Informationen können der Patentanmeldung mit dem Titel "Verfahren zur Ansteuerung einer Ionisierungsvorrichtung", welche am selben Tag und vom selben Anmelder unter dem anmelderseitigen Aktenzeichen 09∼B-110-1 beim deutschen Patient- und Markenamt hinterlegt wurde, entnommen werden. Der Offenbarungsgehalt dieser Patentanmeldung wird vollumfänglich in den Offenbarungsgehalt der vorliegenden Anmeldeschrift aufgenommen.

### Bezugszeichenliste

1. Kraftfahrzeugklimaanlage
2. Gebläse
3. Filter
4. Heizkörper
5. Kraftfahrzeuginnenraum
6. Luftausströmerdüse
7. Ionisierungsmodul
8. Anionen
9. Luftfeitkanal
10. Wandbereich
11. Funktionsgraf
12. Abszisse
13. Ordinate
14. normaler Betriebsmodus
15. Regenerationsmodus
16. Regeneration
17. Raster
18. Metallfolie
19. Außenseite
20. ESD-Folie

## Patentansprüche

1. Luftführungsvorrichtung (6, 9), insbesondere Luftführungskanal (9) und/oder Luftauslassdüse (6), bevorzugt Luftführungsvorrichtung (6, 9) für eine Ionisierungsvorrichtung (7), wobei lokal wirkende Feldkompensationsmittel (18, 20) an der Luftführungsvorrichtung angebracht sind, **dadurch gekennzeichnet, dass** die Feldkompensationsmittel (18, 20) ein regelmäßiges Raster von gleichartig geformten, jeweils zueinander beabstandeten Metallfolien (18) in zueinander versetzter Anordnung oder großflächig auf der Luftführungsvorrichtung angebrachte ESD-Folien (20) aufweisen.

2. Luftführungsvorrichtung (6, 9) nach Anspruch 1, **dadurch gekennzeichnet, dass** die elektrische Leitfähigkeit zumindest einer Metallfolie (18) größer als oder gleich 10⁴ S/m, 10⁵ S/m und/oder 10⁶ S/m ist.

3. Luftführungsvorrichtung (6, 9) nach Anspruche 1 oder 2, **dadurch gekennzeichnet, dass** zumindest zwei lokal wirkende Feldkompensationsmittel (18, 20), insbesondere zumindest zwei Metallfolien (18) voneinander elektrisch entkoppelt sind.

4. Luftführungsvorrichtung (6, 9) nach Anspruch 3, **dadurch gekennzeichnet, dass** die elektrische Entkopplung zumindest teilweise eine Leitfähigkeit von weniger als oder gleich 10⁵ S/m, 10⁴ S/m, 10³ S/m, 10² S/m, 10¹ S/m und/oder 10° S/m aufweist.

5. Luftführungsvorrichtung (6, 9) nach einem der vorangehenden Ansprüche **dadurch gekennzeichnet, dass** zumindest eine ESD-Folie (20) zumindest bereichsweise eine elektrische Leitfähigkeit von kleiner als oder gleich 10⁵ S/m, 10⁴ S/m, 10³ S/m, 10² S/m, 10¹ S/m, 10⁰ S/m, 10⁻¹ S/m, 10⁻² S/m, 10⁻³ S/m, 10⁻⁴ S/m und/oder 10⁻⁵ S/m aufweist.

6. Luftführungsvorrichtung (6, 9) nach einem der vorangehenden Ansprüche, **dadurch gekennzeichnet, dass** zumindest ein lokal wirkendes Feldkompensationsmittel (18, 20), insbesondere zumindest ein elektrisch leitfähiger Materialabschnitt (18) und/oder zumindest eine ESD-Folie (20) selbstklebend ausgeführt ist.

7. Ionisierungsvorrichtung, aufweisend zumindest ein erstes Ionisierungsmittel (7) zur zumindest zeitweisen Freisetzung von Ionen (8) eines ersten Ionentyps, sowie zumindest eine Luftführungsvorrichtung (6, 9) nach einem der Ansprüche 1 bis 6,
wobei die Luftführungsvorrichtung (6, 9) dem Ionisierungsmittel (7) nachgeschaltet angeordnet ist.

8. Ionisierungsvorrichtung nach Anspruch 7 die derart ausgebildet und eingerichtet ist, dass diese zumindest zeitweise in zumindest einem Regenerationsmodus (15, 16) betrieben werden kann.

9. Ionisierungsvorrichtung nach Anspruch 8,
**dadurch gekennzeichnet,**
**dass** in dem zumindest einen Regenerationsmodus (15, 16) zumindest zeitweise Ionen (8) von einem zweiten, vom ersten Ionentyp unterschiedlichen Ionentyp freigesetzt werden und/oder zumindest zeitweise keine Ionen (8), insbesondere keine Ionen vom ersten Ionentyp freigesetzt werden.

10. Klimaanlage (1), insbesondere Kraftfahrzeugklimaanlage, **gekennzeichnet durch** zumindest eine Luftführungsvorrichtung (6, 9) nach einem der Ansprüche 1 bis 6
und/oder zumindest eine Ionisierungsvorrichtung nach einem der Ansprüche 7 bis 9.

## Claims

1. Air conduction device (6, 9), in particular air conduction channel (9) and/or air outlet nozzle (6), preferably air conduction device (6, 9) for an ionisation device (7), wherein locally acting field compensation means (18, 20) are attached to the air conduction device, **characterised in that** the field compensation means (18, 20) have a regular grid of metal foils (18) of the same shape and spaced apart and offset from one another or of ESD foils (20) attached on a large area of the air conduction device.

2. Air conduction device (6, 9) according to claim 1, **characterised in that** the electrical conductivity of at least one metal foil (18) is greater than or equal to 10⁴ S/m, 10⁵ S/m and/or 10⁶ S/m.

3. Air conduction device (6, 9) according to claim 1 or 2, **characterised in that** at least two locally acting field compensation means (18, 20), in particular at least two metal foils (18), are electrically decoupled from one another.

4. Air conduction device (6, 9) according to claim 3, **characterised in that** the electrical decoupling has, at least in part, a conductivity of less than or equal to 10⁵ S/m, 10⁴ S/m, 10³ S/m, 10² S/m, 10¹ S/m and/or 10⁰ S/m.

5. Air conduction device (6, 9) according to one of the preceding claims, **characterised in that** at least one ESD foil (20) has, at least in sections, an electrical conductivity of less than or equal to 10⁵ S/m, 10⁴ S/m, 10³ S/m, 10² S/m, 10¹ S/m, 10⁰ S/m, 10⁻¹ S/m, 10⁻² S/m, 10⁻³ S/m, 10⁻⁴ S/m and/or 10⁻⁵ S/m.

6. Air conduction device (6, 9) according to one of the preceding claims, **characterised in that** at least one locally acting field compensation means (18, 20), in particular at least one electrically conductive material segment (18) and/or at least one ESD foil (20) is designed to be self-adhesive.

7. Ionisation device having at least one first ionisation means (7) for releasing ions (8) of a first ion type, at least at certain times, and having at least one air conduction device (6, 9) according to one of claims 1 through 6, wherein the air conduction device (6, 9) is arranged downstream of the ionisation means (7).

8. Ionisation device according to claim 7 that is designed and configured such that it can be operated in at least one regeneration mode (15, 16), at least at times.

9. Ionisation device according to claim 8, **characterised in that** in the at least one regeneration mode (15, 16), ions (8) of a second type different from the first ion type are released at least at times and/or no ions (8), in particular no ions of the first ion type, are released at least at times.

10. Climate control system (1), in particular motor vehicle climate control system, **characterised by** at least one air conduction device (6, 9) according to one of claims 1 through 6 and/or at least one ionisation device according to one of claims 7 through 9.

## Revendications

1. Dispositif de guidage d'air (6, 9), en particulier conduit de guidage d'air (9) et / ou buse de sortie d'air (6), de préférence dispositif de guidage d'air (6, 9) pour un dispositif d'ionisation (7), où des moyens de compensation de champ (18, 20) agissant localement sont appliqués sur le dispositif de guidage d'air, **caractérisé en ce que** les moyens de compensation de champ (18, 20) présentent une trame régulière de feuilles de métal (18) formées de façon identique, espacées à chaque fois les unes des autres et disposées en étant décalées les unes par rapport aux autres, ou bien présentent des feuilles ESD (20) appliquées sur une grande étendue du dispositif de guidage d'air.

2. Dispositif de guidage d'air (6, 9) selon la revendication 1, **caractérisé en ce que** la conductivité électrique d'au moins une feuille de métal (18) est supérieure ou égale à 10⁴ S/m, à 10⁵ S/m et / ou à 10⁶ S/m.

3. Dispositif de guidage d'air (6, 9) selon la revendication 1 ou 2, **caractérisé en ce qu'**au moins deux moyens de compensation de champ (18, 20) agissant localement, en particulier au moins deux feuilles de métal (18), sont découplés électriquement l'un de l'autre.

4. Dispositif de guidage d'air (6, 9) selon la revendication 3, **caractérisé en ce que** le découplage électrique présente au moins partiellement une conductivité électrique inférieure ou égale à 10⁵ S/m, à 10⁴ S/m, à 10³ S/m, à 10² S/m, 10¹ S/m et / ou à 10° S/m.

5. Dispositif de guidage d'air (6, 9) selon l'une quelconque des revendications précédentes, **caractérisé en ce qu'**au moins une feuille ESD (20) présente au moins partiellement une conductivité électrique inférieure ou égale à 10⁵ S/m, à 10⁴ S/m, à 10³ S/m, à 10² S/m, à 10¹ S/m, à 10⁰ S/m, à 10⁻¹ S/m, à 10⁻² S/m, à 10⁻³ S/m, à 10⁻⁴ S/m et / ou à 10⁻⁵ S/m.

6. Dispositif de guidage d'air (6, 9) selon l'une quelconque des revendications précédentes, **caractérisé en ce qu'**au moins un moyen de compensation de champ (18, 20) agissant localement, en particulier au moins une partie de matière (18) électriquement conductrice et / ou au moins une feuille ESD (20), est réalisé de façon autoadhésive.

7. Dispositif d'ionisation présentant au moins un premier moyen d'ionisation (7) servant à la libération d'ions (8) d'un premier type d'ions, se produisant au moins par moments, ainsi qu'au moins un dispositif de guidage d'air (6, 9) selon l'une quelconque des revendications 1 à 6, où le dispositif de guidage d'air est disposé en aval du moyen d'ionisation (7).

8. Dispositif d'ionisation selon la revendication 7, qui est conçu et agencé de manière telle, que celui-ci puisse être actionné au moins par moments, dans au moins un mode de régénération (15, 16).

9. Dispositif d'ionisation selon la revendication 8, **caractérisé en ce que** dans le mode de régénération (15, 16) au moins au nombre de un sont libérés, au moins par moments, des ions (8) d'un second type d'ions différent du premier type d'ions et / ou, au moins par moments, aucun ion (8) n'est libéré, en particulier aucun ion du premier type d'ions.

10. Système de climatisation (1), en particulier système de climatisation d'un véhicule automobile, **caractérisé par** au moins un dispositif de guidage d'air (6, 9) selon l'une quelconque des revendications 1 à 6, et / ou par au moins un dispositif d'ionisation selon l'une quelconque des revendications 7 à 9.
